# EUROPEAN PATENT APPLICATION

(11) **EP 2 383 579 A1**
(43) Date of publication of application: **02.11.2011**
(21) Application number: 10195593.8
(22) Date of filing: 17.12.2010
(51) Int. Cl.: G01N 33/68, G01N 33/74

(54) **sFlt-1, cardiac troponins and natriuretic peptides in the recognition of therapy with HER-2 inhibitors**

(71) Applicant: F. Hoffmann-La Roche AG, 4070 Basel (CH); Roche Diagnostics GmbH, 68305 Mannheim (DE)
(72) Inventor: The designation of the inventor has not yet been filed
(74) Representative: Huhn, Michael

(57) **Abstract**

The present invention relates to the field of laboratory diagnostics. Specifically, means and methods for the assessing the effects of a therapy with HER-2-inhibitors such as trastuzumab are disclosed.

## Description

The present invention relates to the field of laboratory diagnostics. Specifically, means and methods for the assessing the effects of a therapy with HER-2-inhibitors such as trastuzumab are disclosed.

Trastuzumab is an antibody against human epidermal growth factor receptor 2 (HER-2/neu, also known as Erb-B2). Said antibody is used for the treatment of HER-2-positive breast cancer with great success. There are only few side effects. Trastuzumab is known to reduce the vessel diameter in HER-2-positive tumors and to modulate the induction, expression and release of VEGF (Wen X.F. et al., 2006, Oncogene, 25: 6986-6996; Linderholm, B. et al., 2004, Eur. J. Cancer, 40: 33-42). Trastuzumab is not cardiotoxic to mice and monkeys. Unfortunately, a decrease in the left ventricular ejection fraction (LVEF) can occasionally be observed after trastuzumab therapy in humans. This is, however, considered to be a temporary phenomenon without evidence of long-term cardiotoxic side effects (Ewer M.S. et al., 2005, J. Clin. Oncol., 23: 7820-7826) and was found to be reversible after the discontinuation of trastuzumab therapy. Studies in mice indicate that HER-2 is expressed by cardiomyocytes and is responsible for myocardial function and survival (Ozcelik C., et al., 2002, Proc. Natl. Acad. Sci. USA, 99: 8880-8885).

Troponins represent structural proteins of myocytes that are released upon myocyte death. Troponins have been used for the diagnosis of myocardial infarction (Reichlin et al., 2009, NEJM, 361: 858-867; Keller et al., 2009, NEJM, 361: 868-877). With the development of high sensitivity troponin tests persistent elevations of troponins have been noted that correlate with the extent of heart failure as with coronary artery disease (EP 07 114 174).

Soluble fms-like tyrosine kinase (sFlT1) is a receptor for vascular endothelial growth factor (VEGF) and placental growth factor (PlGF). sFlt1 has been shown to be released into the circulation under ischemic conditions such as acute coronary syndrome and myocardial infarction (EP10156911.9). Under these conditions rapid increases and decreases of the levels of sFlT1 related to symptoms of ischemia such as chest pain have been noted. In cardiovascular disease increase of sFlT1 may be followed by cardiac necrosis as indicated by an increase in troponin, both of which can be detected in serum or plasma. Increased blood levels of sFlT1 are, however, not specific for cardiac complications and may also occur during ischemia in other parts of the body.

As mentioned above, cardiotoxic effects of trastuzumab (decrease in LVEF) appear to be fully reversible, without leaving long-term cardiotoxic side effects in cases when treatment is remitted or only continued for an appropriate period in time. The decision when trastuzumab therapy is remitted, continued or modified is left to the discretion of the physician who has to base his decision on more or less subjective criteria. Cardiotoxic side effects of HER 2 inhibitors are currently diagnosed using echocardiography, preferably by determining the left ventricular ejection fraction (LVEF). However, methods for detecting diastolic dysfunction using Doppler echocardiography can also be used.

To date, there is no method or device at hand which permits to monitor a patient receiving HER-2 inhibitor treatment with respect to possible cardiotoxic effects, and to help the physician decide if or when HER-2 inhibitor therapy is to be modified or remitted.

The problem underlying the present invention can be seen as the provision of methods and kits allowing the early diagnosis or detection of undesired side-effects of trastuzumab. This diagnosis or detection should allow the monitoring of the response of the cancer to treatment and permit or give guidance in respect to a decision of remittance, modification or continuation of the treatment.

The technical problem is solved by the embodiments characterized in the claims and herein below.

The present invention relates to a method for assessing the effects of a therapy with a HER-2-inhibitor based on the determination of sFlt1 or a variant thereof and a cardiac troponin or a variant thereof in a sample of a patient and the comparison the measured amounts of said markers with reference amounts.

It is also provided a method for assessing the effects of a therapy with a HER-2-inhibitor, comprising the steps of
a) determining the amount of sFlt-1 or a variant thereof and a cardiac troponin or a variant thereof in a sample of the patient; and
b) comparing the measured amounts of sFIT-1 or the variant thereof and the cardiac troponin or the variant thereof with reference amounts;
whereby effects of the therapy with a HER-2-inhibitor are assessed based on the results of the comparison performed in step b).

Preferably, the method of the present invention comprises the steps of a) determining the amount of sFlt-1 or a variant thereof and a cardiac troponin or a variant thereof in a sample of the patient; b) comparing the measured amounts of sFIT-1 or the variant thereof and the cardiac troponin or the variant thereof with reference amounts; and c) assessing the effects of the therapy with a HER-2-inhibitor based on the results obtained in step b).

Accordingly, the present invention relates to a method for assessing the effects of a therapy with a HER-2-inhibitor, comprising the steps of
a) determining the amount of sFlt-1 or a variant thereof and a cardiac troponin or a variant thereof in a sample of the patient;
b) comparing the measured amounts of sFlT-1 or the variant thereof and the cardiac troponin or the variant thereof with reference amounts; and
c) assessing the effects of the therapy with a HER-2-inhibitor based on the results obtained in step b).

The method of the present invention is, preferably, an in vitro method. Moreover, it may comprise steps in addition to those explicitly mentioned above including sample pretreatments or evaluation of the results obtained by the method. The method may be carried out manually and/or assisted by automation. Preferably, steps (a), (b) and/or (c) may in total or in part be assisted by automation including suitable robotic and sensory equipment for the determination in step (a) and/or a computer-implemented comparison under steps (b) and/or (c).

The term "sample" refers to a sample of a body fluid, to a sample of separated cells or to a sample from a tissue or an organ. Samples of body fluids can be obtained by well known techniques and include, preferably, samples of blood, plasma, serum, or urine, more preferably, samples of blood, plasma or serum. Tissue or organ samples may be obtained from any tissue or organ by, e.g., biopsy. Separated cells may be obtained from the body fluids or the tissues or organs by separating techniques such as centrifugation or cell sorting. Preferably, cell-, tissue- or organ samples are obtained from those cells, tissues or organs which express or produce the peptides referred to herein.

The patient is, preferably, a human. Preferably, the patient is apparently healthy with respect to diseases and disorders other than cancer. In the context of the present invention, the term "apparently healthy" is known to the person skilled in the art and refers to a subject which does not show obvious signs or symptoms of an underlying disease. The subject, thus, may suffer from said disease, but preferably does not show obvious signs or symptoms such that said disease can be diagnosed or assessed without detailed diagnostic examination by a physician. In particular, the diagnosis by a specialist would be required to diagnose the disease other than cancer in the apparently healthy subject not showing obvious symptoms of the disease. Cardiac diseases are, preferably, stable heart diseases, more preferably heart failure, acquired heart valve disorders, coronary artery disease and stable angina pectoris. Also preferably the patient is healthy with respect to diseases other than cancer and cardiac diseases.

The person skilled in the art is aware that the concentrations cited in the present application for the cardiac troponins (troponin T or a variant thereof and troponin I or a variant thereof), NT-proANP or a variant thereof and - to a lesser extent - for sFLT-1 or a variant thereof, HGF or a variant thereof, H-FABP or a variant thereof and myoglobin or a variant thereof may not apply for patients suffering from impaired renal function, preferably patients suffering from renal failure, in particular patients suffering from chronic and end stage renal failure. In a preferred embodiment of the present invention, patients suffering from impaired renal function, preferably patients suffering from renal failure, in particular patients suffering from chronic and end stage renal failure are not comprised in (excluded from) the methods of the present invention. In another preferred embodiment, patients with renal hypertension are not comprised in (excluded from) the methods of the present invention. Preferably, the "subject" as used herein excludes patients suffering from impaired renal function, preferably patients suffering from renal failure, in particular patients suffering from chronic and end stage renal failure, more preferably patients with renal hypertension, most preferably all of the patients suffering from one of the diseases and conditions mentioned in this sentence. In this context, "renal failure" is regarded as an impaired glomerular filtration rate (GFR) lying below the usual ranges of 60 to 120 ml/min, preferably below 60 ml/min. Furthermore, the normal kidney function may be assessed using the determination of creatinine in serum. Preferably, a normal kidney function is assumed if the creatinine value is within the normal range of the test method. Also preferably, Cystatin C (with values within the normal range) is measured in serum. Subjects with impaired renal function show higher levels of troponin I and troponin T than those cited above, due to an impaired clearance of the peptide. The levels vary with the severity of the renal impairment.

The patient according to the present invention suffers from cancer. The term "cancer" refers to any type of solid or hematological cancer, where the cancer cells overexpress HER-2. The cancer is, preferably lung cancer, gastric cancer, colon cancer, breast cancer, ovarian cancer, endometrial cancer, testicular cancer, prostrate cancer, skin melanoma, hepatic carcinoma, pancreatic carcinoma, kidney carcinoma, bladder carcinoma, retinoblastoma, neuroblastoma, glioblastoma, osteosarcoma, Ewing's sarcoma, Hodgkin lymphoma, non-Hodgkin lymphoma and leukemia. More preferably, the cancer is breast cancer, gastric cancer or uterine serous carcinoma. Most preferably, the cancer is breast cancer.

The term "HER-2-inhibitor" refers to any substance that negatively interferes with the activity of the membrane-bound receptor tyrosine kinase HER-2/neu that is also known as ErbB2. Said kinase is a receptor belonging to the epidermal growth factor family. Preferably, negative interference with the activity of the receptor relates to reducing the expression of said receptor or its activity. Preferably, the expression of HER-2 is reduced on the transcriptional level using antisense-technologies or ribozymes. The activity of the receptor is, preferably, reduced by competitive or non-competitive inhibition. Both types of inhibition are mediated by the binding of one or more molecules to the receptor. In the case of competitive inhibition the inhibitor binds to ligand binding site of the receptor and thereby prevents the ligand from binding. Since the inhibitor does not activate the receptor, the activation of the receptor by its ligand is reduced. In the case of non-competitive inhibition the inhibitor binds to a part of the receptor which is not the ligand binding site. By changing the conformation of the receptor, the inhibitor prevents the ligand from binding and/or inhibits the catalytic activity of the receptor, in the case of HER-2 the phosphorylation of tyrosine, upon binding of the ligand. The HER-2-inhibitor is, preferably, a peptide or a small molecule. More preferably, the peptide is an antibody.

Preferably, the antibody is a monoclonal antibody, a polyclonal antibody, a single chain antibody, a human or humanized antibody or a fragment thereof. Also comprised as antibodies by the present invention are a bispecific antibody, a synthetic antibody, an antibody fragment, such as Fab, Fv or scFv fragments etc., or a chemically modified derivative of any of these. The antibody is, most preferably, trastuzumab or an antibody fragment, such as a Fab, Fv or scFv fragment etc. of trastuzumab, or a chemically modified derivative of any of these.

In a preferred embodiment of the present invention the "therapy with a HER-2-inhibitor" is a monotherapy with a HER-2-inhibitor.

In a further preferred embodiment of the present invention the "therapy with a HER-2-inhibitor" is a combination therapy comprising at least one further pharmaceutical in addition to the HER-2-inhibitor.

In a preferred embodiment of the invention, said combination therapy does not comprise an anthracyclin. In yet another preferred embodiment of the present invention the "therapy with a HER-2-inhibitor" is a combination therapy comprising at least the HER-2-inhibitor and an anthracyclin.

The term "assessing the effects of a therapy with a HER-2-inhibitor" refers to the process of determining whether (i) said therapy achieves the desired effect and (ii) whether said therapy causes undesired side-effects. For the assessment of the effects of the therapy with the HER-2-inhibitor the sample is, preferably, taken about 2 weeks, about 4 weeks or about 6 weeks after the initiation of said therapy. The term "about" in this context refers to +/-5% of a given value.

In a preferred embodiment the present invention relates to a method for monitoring the therapy with a HER-2-inhibitor.

Accordingly, the present invention relates to a method for monitoring therapy with a HER-2-inhibitor, comprising the steps of
a) repeatedly determining the amount of sFlt-1 or a variant thereof and a cardiac troponin or a variant thereof in a sample of the patient;
b) comparing the measured amounts of sFIT-1 or the variant thereof and the cardiac troponin or the variant thereof with reference amounts; and
c) assessing the effects of the therapy with a HER-2-inhibitor based on the results obtained in step b).

The term "monitoring", preferably, refers to a process of repeated (more than one) samplings from the patient. Preferably, samples are taken in intervals of about 1 week, about 2 weeks, about 3 weeks, about 4 weeks, about 5 weeks or about 6 weeks, more preferably about 4 weeks. Preferably, the monitoring continues as long as the patient is treated with the HER-2-inhibitor. The term "about" in this context refers to +/- 5% of a given value. For monitoring the therapy with a HER-2-inhibitor the amounts of the markers of the present invention determined in each of said samples can be compared to reference amounts determined in a sample taken from the same patient before the initiation of therapy. It is also possible to compare the amounts of the markers of the present invention determined in a sample to the amounts in one or more samples taken before the aforementioned sample but after the onset of therapy. Thus, the effects of the therapy with a HER-2-inhibitor can be observed during a longer period of treatment and the onset of undesired side-effects or changes of the desired effect can be readily detected.

As will be understood by those skilled in the art, the results of assessing or monitoring the therapy with a HER-2-inhibitor are usually not intended to be correct for 100% of the patients to be assessed or monitored. The term, however, requires that a statistically significant portion of patients can be diagnosed with respect to response of the tumor to treatment and/or undesired side-effects. Whether a portion is statistically significant can be determined without further ado by the person skilled in the art using various well known statistic evaluation tools, e.g., determination of confidence intervals, p-value determination, Student's t-test, Mann-Whitney test, etc.. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1, 0.05, 0.01, 0.005, or 0.0001.

The desired effect of HER-2-therapy in cancer-patients is the successful treatment of the cancer. In solid cancers a successful treatment of the cancer is, preferably, indicated by response of at least one tumor in the patient to said therapy. Preferably, a response of a tumor to therapy with a HER-2-inhibitor is indicated by the death of at least a part of the tumor cells and the concomitant reduction of tumor size. Because dead tumor cells are not always removed immediately after their death, a tumor may respond to treatment even though an immediate reduction of tumor size cannot be observed. Therefore, a reduction of the metabolic activity in at least a part of the tumor cells of at least one tumor in the patient is also preferred as an indicator of tumor response to therapy with a HER-2-inhibitor. Also preferably, a response of the tumor to therapy is indicated by a prolonged time until tumor progression. More preferably, a response of the tumor to therapy with a HER-2-inhibitor is indicated by a reduction of tumor size. Most preferably, a response of the tumor to treatment is indicated by a size reduction of the tumor by at least about 40 %, by at least about 50 % or by at least about 60 % within about 1 month, about 2 months, about 3 months or about 4 months after the onset of therapy with a HER-2-inhibitor. The term "about" in this context refers to +/- 5% of a given measurement.

Size reduction of a tumor is, preferably, observed with imaging methods. Preferred imaging methods are x-ray with or without use of a contrast agent, computed tomography, sonography and magnetic resonance imaging. Reduced metabolic activity of tumor cells and reduced tumor size are, preferably, indicated by positron emission tomography - computed tomography (PET-CT) or positron emission tomography - magnetic resonance imaging (PET-MRI). Fluorine-18 Fluorodeoxyglucose (FDG) is a preferred radiotracer to be used for PET. FDG is taken up by the cells without being metabolized, thus it accumulates. Different degrees of accumulation in different tissues are a measure for different metabolic activity of these tissues. Thus, the metabolic activity of a tumor can be determined with FDG as a tracer.

Undesired side-effects of therapy with a HER-2-inhibitor are those consequences of said therapy that are detrimental to the health of the patient. Undesired side-effects of therapy with a HER-2-inhibitor are, mainly, cardiotoxic side effects, more preferably heart failure.

Heart failure is, preferably, systolic or diastolic heart failure. Also preferably, it may be congestive heart failure.

The term "soluble (s)FLT-1" as used herein refers to polypeptide which is a soluble form of the VEGF receptor FLT1. It was identified in conditioned culture medium of human umbilical vein endothelial cells. The endogenous soluble FLT1 (sFLT1) receptor is chromatographically and immunologically similar to recombinant human sFLT1 and binds VEGF with a comparable high affinity. Human sFLT1 is shown to form a VEGF-stabilized complex with the extracellular domain of KDR/Flk-1 in vitro. Preferably, sFLT1 refers to human sFLT1. More preferably, human sFLT1 can be deduced from the amino acid sequence of Flt-1 as shown in Genebank accession number P17948, GI: 125361. An amino acid sequence for mouse sFLT1 is shown in Genebank accession number BAA24499.1, GI: 2809071.

The term "cardiac Troponin" refers to all Troponin isoforms expressed in cells of the heart and, preferably, the subendocardial cells. These isoforms are well characterized in the art as described, e.g., in Anderson 1995, Circulation Research, vol. 76, no. 4: 681-686 and Ferrieres 1998, Clinical Chemistry, 44: 487-493. Preferably, cardiac Troponin refers to Troponin T and/or Troponin I, and, most preferably, to Troponin T. It is to be understood that isoforms of Troponins may be determined in the method of the present invention together, i.e. simultaneously or sequentially, or individually, i.e. without determining the other isoform at all. Amino acid sequences for human Troponin T and human Troponin I are disclosed in Anderson, loc cit and Ferrieres 1998, Clinical Chemistry, 44: 487-493.

Preferably the biological property of troponin I and its variant is the ability to inhibit actomyosin ATPase or to inhibit angiogenesis in vivo and in vitro, which may e.g. be detected based on the assay described by Moses et al. 1999 PNAS USA 96 (6): 2645-2650). Preferably the biological property of troponin T and its variant is the ability to form a complex with troponin C and I, to bind calcium ions or to bind to tropomyosin, preferably if present as a complex of troponin C, I and T or a complex formed by troponin C, troponin I and a variant of troponin T.

The term "variant" encompasses also variants of the specific peptides of the present application. Such variants have at least the same essential biological and immunological properties as the specific cardiac Troponins and sFlT1. In particular, they share the same essential biological and immunological properties if they are detectable by the same specific assays referred to in this specification, e.g., by ELISA Assays using polyclonal or monoclonal antibodies specifically recognizing the said cardiac Troponins and sFlT1. Moreover, it is to be understood that a variant as referred to in accordance with the present invention shall have an amino acid sequence which differs due to at least one amino acid substitution, deletion and/or addition wherein the amino acid sequence of the variant is still, preferably, at least about 50%, at least about 60%, at least about 70%, at least about 80%, at least about 85%, at least about 90%, at least about 92%, at least about 95%, at least about 97%, at least about 98%, or at least about 99% identical with the amino sequence of the specific cardiac Troponin or sFlT1, preferably over the entire length of the specific peptide. Variants may be allelic variants or any other species specific homologs, paralogs, or orthologs. Moreover, the variants referred to herein include fragments of the specific cardiac Troponins or sFlT1 or the aforementioned types of variants as long as these fragments have the essential immunological and biological properties as referred to above. Preferably the biological property of troponin I and its variant is the ability to inhibit actomyosin ATPase or to inhibit angiogenesis in vivo and in vitro, which may e.g. be detected based on the assay described by Moses et al. 1999 PNAS USA 96 (6): 2645-2650). Preferably the biological property of troponin T and its variant is the ability to form a complex with troponin C and I, to bind calcium ions or to bind to tropomyosin, preferably if present as a complex of troponin C, I and T or a complex formed by troponin C, troponin I and a variant of troponin T. The endogenous soluble FLT1 (sFLT1) receptor binds VEGF with high affinity. Preferably, human sFLT1 is shown to form a VEGF-stabilized complex with the extracellular domain of KDR/Flk-1 in vitro. Thus, the variants shall be recognizable by the aforementioned means or ligands used for determination of the amount of the cardiac troponins or sFlT1. Such fragments may be, e.g., degradation products of the peptides of the present invention. Further included are variants which differ due to posttranslational modifications such as phosphorylation or myristylation.

Determining the amount of sFlT1 or the amount of a cardiac troponin, preferably troponin T, or any other peptide or polypeptide or protein referred to in this specification relates to measuring the amount or concentration, preferably semi-quantitatively or quantitatively. The terms polypeptide and protein are used interchangeable throughout this application. Measuring can be done directly or indirectly. Direct measuring relates to measuring the amount or concentration of the peptide or polypeptide based on a signal which is obtained from the peptide or polypeptide itself and the intensity of which directly correlates with the number of molecules of the peptide present in the sample. Such a signal - sometimes referred to herein as intensity signal -may be obtained, e.g., by measuring an intensity value of a specific physical or chemical property of the peptide or polypeptide. Indirect measuring includes measuring of a signal obtained from a secondary component (i.e. a component not being the peptide or polypeptide itself) or a biological read out system, e.g., measurable cellular responses, ligands, labels, or enzymatic reaction products.

In accordance with the present invention, determining the amount of a peptide or polypeptide can be achieved by all known means for determining the amount of a peptide in a sample. Said means comprise immunoassay devices and methods which may utilize labelled molecules in various sandwich, competition, or other assay formats. Said assays will develop a signal which is indicative for the presence or absence of the peptide or polypeptide. Moreover, the signal strength can, preferably, be correlated directly or indirectly (e.g. reverse- proportional) to the amount of polypeptide present in a sample. Further suitable methods comprise measuring a physical or chemical property specific for the peptide or polypeptide such as its precise molecular mass or NMR spectrum. Said methods comprise, preferably, biosensors, optical devices coupled to immunoassays, biochips, analytical devices such as mass- spectrometers, NMR- analyzers, or chromatography devices. Further, methods include micro-plate ELISA-based methods, fully-automated or robotic immunoassays (available for example on ElecsysTM analyzers), CBA (an enzymatic Cobalt Binding Assay, available for example on Roche-HitachiTM analyzers), and latex agglutination assays (available for example on Roche-HitachiTM analyzers).

Preferably, determining the amount of a peptide or polypeptide comprises the steps of (a) contacting a cell capable of eliciting a cellular response the intensity of which is indicative of the amount of the peptide or polypeptide with the said peptide or polypeptide for an adequate period of time, (b) measuring the cellular response. For measuring cellular responses, the sample or processed sample is, preferably, added to a cell culture and an internal or external cellular response is measured. The cellular response may include the measurable expression of a reporter gene or the secretion of a substance, e.g. a peptide, polypeptide, or a small molecule. The expression or substance shall generate an intensity signal which correlates to the amount of the peptide or polypeptide.

Also preferably, determining the amount of a peptide or polypeptide comprises the step of measuring a specific intensity signal obtainable from the peptide or polypeptide in the sample. As described above, such a signal may be the signal intensity observed at an m/z variable specific for the peptide or polypeptide observed in mass spectra or a NMR spectrum specific for the peptide or polypeptide.

Determining the amount of a peptide or polypeptide may, preferably, comprise the steps of (a) contacting the peptide with a specific ligand, (b) (optionally) removing non-bound ligand, (c) measuring the amount of bound ligand. The bound ligand will generate an intensity signal. Binding according to the present invention includes both covalent and non-covalent binding. A ligand according to the present invention can be any compound, e.g., a peptide, polypeptide, nucleic acid, or small molecule, binding to the peptide or polypeptide described herein. Preferred ligands include antibodies, nucleic acids, peptides or polypeptides such as receptors or binding partners for the peptide or polypeptide and fragments thereof comprising the binding domains for the peptides, and aptamers, e.g. nucleic acid or peptide aptamers. Methods to prepare such ligands are well-known in the art. For example, identification and production of suitable antibodies or aptamers is also offered by commercial suppliers. The person skilled in the art is familiar with methods to develop derivatives of such ligands with higher affinity or specificity. For example, random mutations can be introduced into the nucleic acids, peptides or polypeptides. These derivatives can then be tested for binding according to screening procedures known in the art, e.g. phage display. Antibodies as referred to herein include both polyclonal and monoclonal antibodies, as well as fragments thereof, such as Fv, Fab and F(ab)2 fragments that are capable of binding antigen or hapten. The present invention also includes single chain antibodies and humanized hybrid antibodies wherein amino acid sequences of a non-human donor antibody exhibiting a desired antigen-specificity are combined with sequences of a human acceptor antibody. The donor sequences will usually include at least the antigen-binding amino acid residues of the donor but may comprise other structurally and/or functionally relevant amino acid residues of the donor antibody as well. Such hybrids can be prepared by several methods well known in the art. Preferably, the ligand or agent binds specifically to the peptide or polypeptide. Specific binding according to the present invention means that the ligand or agent should not bind substantially to ("cross-react" with) another peptide, polypeptide or substance present in the sample to be analyzed. Preferably, the specifically bound peptide or polypeptide should be bound with at least 3 times higher, more preferably at least 10 times higher and even more preferably at least 50 times higher affinity than any other relevant peptide or polypeptide. Non-specific binding may be tolerable, if it can still be distinguished and measured unequivocally, e.g. according to its size on a Western Blot, or by its relatively higher abundance in the sample. Binding of the ligand can be measured by any method known in the art. Preferably, said method is semi-quantitative or quantitative. Suitable methods are described in the following.

First, binding of a ligand may be measured directly, e.g. by NMR or surface plasmon resonance.

Second, if the ligand also serves as a substrate of an enzymatic activity of the peptide or polypeptide of interest, an enzymatic reaction product may be measured (e.g. the amount of a protease can be measured by measuring the amount of cleaved substrate, e.g. on a Western Blot). Alternatively, the ligand may exhibit enzymatic properties itself and the "ligand/peptide or polypeptide" complex or the ligand which was bound by the peptide or polypeptide, respectively, may be contacted with a suitable substrate allowing detection by the generation of an intensity signal. For measurement of enzymatic reaction products, preferably the amount of substrate is saturating. The substrate may also be labelled with a detectable label prior to the reaction. Preferably, the sample is contacted with the substrate for an adequate period of time. An adequate period of time refers to the time necessary for a detectable, preferably measurable, amount of product to be produced. Instead of measuring the amount of product, the time necessary for appearance of a given (e.g. detectable) amount of product can be measured.

Third, the ligand may be coupled covalently or non-covalently to a label allowing detection and measurement of the ligand. Labelling may be done by direct or indirect methods. Direct labelling involves coupling of the label directly (covalently or non-covalently) to the ligand. Indirect labelling involves binding (covalently or non-covalently) of a secondary ligand to the first ligand. The secondary ligand should specifically bind to the first ligand. Said secondary ligand may be coupled with a suitable label and/or be the target (receptor) of tertiary ligand binding to the secondary ligand. The use of secondary, tertiary or even higher order ligands is often used to increase the signal. Suitable secondary and higher order ligands may include antibodies, secondary antibodies, and the well-known streptavidin-biotin system (Vector Laboratories, Inc.). The ligand or substrate may also be "tagged" with one or more tags as known in the art. Such tags may then be targets for higher order ligands. Suitable tags include biotin, digoxygenin, His-Tag, Glutathion-S-Transferase, FLAG, GFP, myc-tag, influenza A virus haemagglutinin (HA), maltose binding protein, and the like. In the case of a peptide or polypeptide, the tag is preferably at the N-terminus and/or C-terminus. Suitable labels are any labels detectable by an appropriate detection method. Typical labels include gold particles, latex beads, acridan ester, luminol, ruthenium, enzymatically active labels, radioactive labels, magnetic labels ("e.g. magnetic beads", including paramagnetic and superparamagnetic labels), and fluorescent labels. Enzymatically active labels include e.g. horseradish peroxidase, alkaline phosphatase, beta-Galactosidase, Luciferase, and derivatives thereof. Suitable substrates for detection include di-amino-benzidine (DAB), 3,3'-5,5'-tetramethylbenzidine, NBT-BCIP (4-nitro blue tetrazolium chloride and 5-bromo-4-chloro-3-indolyl-phosphate, available as ready-made stock solution from Roche Diagnostics), CDP-Star™ (Amersham Biosciences), ECF™ (Amersham Biosciences). A suitable enzyme-substrate combination may result in a coloured reaction product, fluorescence or chemoluminescence, which can be measured according to methods known in the art (e.g. using a light-sensitive film or a suitable camera system). As for measuring the enzymatic reaction, the criteria given above apply analogously. Typical fluorescent labels include fluorescent proteins (such as GFP and its derivatives), Cy3, Cy5, Texas Red, Fluorescein, and the Alexa dyes (e.g. Alexa 568). Further fluorescent labels are available e.g. from Molecular Probes (Oregon). Also the use of quantum dots as fluorescent labels is contemplated. Typical radioactive labels include 35S, 1251, 32P, 33P and the like. A radioactive label can be detected by any method known and appropriate, e.g. a light-sensitive film or a phosphor imager. Suitable measurement methods according the present invention also include precipitation (particularly immunoprecipitation), electrochemiluminescence (electro-generated chemiluminescence), RIA (radioimmunoassay), ELISA (enzyme-linked immunosorbent assay), sandwich enzyme immune tests, electrochemiluminescence sandwich immunoassays (ECLIA), dissociation-enhanced lanthanide fluoro immuno assay (DELFIA), scintillation proximity assay (SPA), turbidimetry, nephelometry, latex-enhanced turbidimetry or nephelometry, or solid phase immune tests. Further methods known in the art (such as gel electrophoresis, 2D gel electrophoresis, SDS polyacrylamid gel electrophoresis (SDS-PAGE), Western Blotting, and mass spectrometry), can be used alone or in combination with labelling or other detection methods as described above.

The amount of a peptide or polypeptide may be, also preferably, determined as follows: (a) contacting a solid support comprising a ligand for the peptide or polypeptide as specified above with a sample comprising the peptide or polypeptide and (b) measuring the amount peptide or polypeptide which is bound to the support. The ligand, preferably chosen from the group consisting of nucleic acids, peptides, polypeptides, antibodies and aptamers, is preferably present on a solid support in immobilized form. Materials for manufacturing solid supports are well known in the art and include, inter alia, commercially available column materials, polystyrene beads, latex beads, magnetic beads, colloid metal particles, glass and/or silicon chips and surfaces, nitrocellulose strips, membranes, sheets, duracytes, wells and walls of reaction trays, plastic tubes etc. The ligand or agent may be bound to many different carriers. Examples of well-known carriers include glass, polystyrene, polyvinyl chloride, polypropylene, polyethylene, polycarbonate, dextran, nylon, amyloses, natural and modified celluloses, polyacrylamides, agaroses, and magnetite. The nature of the carrier can be either soluble or insoluble for the purposes of the invention. Suitable methods for fixing/immobilizing said ligand are well known and include, but are not limited to ionic, hydrophobic, covalent interactions and the like. It is also contemplated to use "suspension arrays" as arrays according to the present invention (Nolan 2002, Trends Biotechnol. 20(1):9-12). In such suspension arrays, the carrier, e.g. a microbead or microsphere, is present in suspension. The array consists of different microbeads or microspheres, possibly labelled, carrying different ligands. Methods of producing such arrays, for example based on solid-phase chemistry and photo-labile protective groups, are generally known (US 5,744,305).

Preferably, the amount of a cardiac troponin and sFlT1 and, as the case may be, the amounts of other peptides measured in the context of the present invention are determined in a blood sample, e.g., a serum or plasma sample, obtained from a subject as defined in the present invention. Preferably, such a determination is done by ELISA. Such a determination of sFlT1 by ELISA can be done, e.g., by using the ELECSYS sFlT1 test by Roche Diagnostics, Mannheim, Germany. The amount of troponin T can be determined by the COBAS assay, Roche Diagnostics Mannheim, Germany.

The term "amount" as used herein encompasses the absolute amount (e.g., of sFlT1 or a cardiac troponin), the relative amount or concentration (e.g, of sFlT1 or a cardiac troponin) as well as any value or parameter which correlates thereto. Such values or parameters comprise intensity signal values from all specific physical or chemical properties obtained from the said peptides by direct measurements, e.g., intensity values in mass spectra or NMR spectra. Moreover, encompassed are all values or parameters which are obtained by indirect measurements specified elsewhere in this description, e.g., expression amounts determined from biological read out systems in response to the peptides or intensity signals obtained from specifically bound ligands. It is to be understood that values correlating to the aforementioned amounts or parameters can also be obtained by all standard mathematical operations.

The term "comparing" as used herein encompasses comparing the amount of the peptide, polypeptide, protein comprised by the sample to be analyzed with an amount of a reference source specified elsewhere in this description. It is to be understood that comparing as used herein refers to a comparison of corresponding parameters or values, e.g., an absolute amount is compared to an absolute reference amount while a concentration is compared to a reference concentration or an intensity signal obtained from a test sample is compared to the same type of intensity signal of a reference sample. The comparison referred to in step (b) of the method of the present invention may be carried out manually or computer assisted. For a computer assisted comparison, the value of the determined amount may be compared to values corresponding to references which are stored in a database by a computer program. The computer program may further evaluate the result of the comparison, i.e. automatically provide the desired assessment in a suitable output format. Based on the comparison of the amount(s) determined in step a) to reference amount(s), it is possible to diagnose ischemia, reversible cardiac dysfunction and/or non reversible cardiac injury in said subject. It is to be understood that amounts of sFlT1 or a cardiac troponin as determined in step (a) of the methods of the presents invention are compared in step (b) to reference amounts for sFlT1 or a cardiac troponin as specified elsewhere in this application.

In general, for determining the respective amounts/amounts or amount ratios allowing to establish the desired diagnosis in accordance with the respective embodiment of the present invention, ("threshold", "reference amount"), the amount/amount(s) or amount ratios of the respective peptide or peptides are determined in appropriate patient groups. The term "reference amount" with respect to a cardiac troponin refers, preferably, to an amount of the cardiac troponin representing the upper level of normal found in a patient without cardiotoxic side-effects. The term "reference amount", preferably, refers to the amount of a cardiac troponin that allows the differentiation between a patient with and without cardiotoxic side-effects. With respect to sFlT1 the term "reference amount" with refers, preferably, to an amount of the sFlT1 representing the upper level of normal found in a patient without cardiotoxic side-effects and/or without a response of the tumor to treatment. The term "reference amount", preferably, refers to the amount of sFlT1 that allows the differentiation between a patient with and without cardiotoxic side-effects and/or a response of the tumor to treatment. The upper limit of normal for a cardiac troponin or sFIT-1 is, preferably, defined by the 50th percentile, 55th percentile, 60th percentile, 65th percentile, 70th percentile, 75th percentile, 80th percentile, 85th percentile, 90th percentile, 90th percentile or 99th percentile as the case may be.

According to the diagnosis to be established, the patient group may, for example, comprise only healthy individuals, or may comprise healthy individuals and individuals suffering from the pathopysiological state which is to be determined, or may comprise only individuals suffering from the pathopysiological state which is to be determined, or may comprise individuals suffering from the various pathophysiological states to be distinguished, by the respective marker(s) using validated analytical methods. In the context of monitoring the course of a disease the groups are formed, preferably, by patients whose disease worsens and by patients whose disease improves over the course of observation. The results which are obtained are collected and analyzed by statistical methods known to the person skilled in the art. The obtained threshold values are then established in accordance with the desired probability of suffering from the disease and linked to the particular threshold value. For example, it may be useful to choose the median value, the 60th, 70th, 80th, 90th, 95th or even the 99th percentile of the healthy and/or non-healthy patient collective, in order to establish the threshold value(s), reference value(s) or amount ratios.

Consequently, threshold amounts for a cardiac troponin can be derived by determining the amount of the cardiac troponin in individuals that did not suffer from cardiotoxic side-effects of the therapy with a HER-2-inhibitor. A reference amount for sFlT1 is, preferably, derived from a group of patients without cardiotoxic side effects of said therapy and/or without a response of the tumor to treatment.

A reference value of a diagnostic marker can be established, and the amount of the marker in a patient sample can simply be compared to the reference value. The sensitivity and specificity of a diagnostic and/or prognostic test depends on more than just the analytical "quality" of the test-they also depend on the definition of what constitutes an abnormal result. In practice, Receiver Operating Characteristic curves, or "ROC" curves, are typically calculated by plotting the value of a variable versus its relative frequency in "normal" and "disease" populations. For any particular marker of the invention, a distribution of marker amounts for subjects with and without a disease will likely overlap. Under such conditions, a test does not absolutely distinguish normal from disease with 100% accuracy, and the area of overlap indicates where the test cannot distinguish normal from disease. A threshold is selected, above which (or below which, depending on how a marker changes with the disease) the test is considered to be abnormal and below which the test is considered to be normal. The area under the ROC curve is a measure of the probability that the perceived measurement will allow correct identification of a condition. ROC curves can be used even when test results don't necessarily give an accurate number. As long as one can rank results, one can create an ROC curve. For example, results of a test on "disease" samples might be ranked according to degree (say 1=low, 2=normal, and 3=high). This ranking can be correlated to results in the "normal" population, and a ROC curve created. These methods are well known in the art. See, e.g., Hanley et al, Radiology 143: 29-36 (1982).

In certain embodiments, markers and/or marker panels are selected to exhibit at least about 70% sensitivity, more preferably at least about 80% sensitivity, even more preferably at least about 85% sensitivity, still more preferably at least about 90% sensitivity, and most preferably at least about 95% sensitivity, combined with at least about 70% specificity, more preferably at least about 80% specificity, even more preferably at least about 85% specificity, still more preferably at least about 90% specificity, and most preferably at least about 95% specificity. In particularly preferred embodiments, both the sensitivity and specificity are at least about 75%, more preferably at least about 80%, even more preferably at least about 85%, still more preferably at least about 90%, and most preferably at least about 95%. The term "about" in this context refers to +/- 5% of a given measurement.

In other embodiments, a positive likelihood ratio, negative likelihood ratio, odds ratio, or hazard ratio is used as a measure of a test's ability to predict risk or diagnose a disease. In the case of a positive likelihood ratio, a value of 1 indicates that a positive result is equally likely among subjects in both the "diseased" and "control" groups; a value greater than 1 indicates that a positive result is more likely in the diseased group; and a value less than 1 indicates that a positive result is more likely in the control group. In the case of a negative likelihood ratio, a value of 1 indicates that a negative result is equally likely among subjects in both the "diseased" and "control" groups; a value greater than 1 indicates that a negative result is more likely in the test group; and a value less than 1 indicates that a negative result is more likely in the control group. In certain preferred embodiments, markers and/or marker panels are preferably selected to exhibit a positive or negative likelihood ratio of at least about 1.5 or more or about 0.67 or less, more preferably at least about 2 or more or about 0.5 or less, still more preferably at least about 5 or more or about 0.2 or less, even more preferably at least about 10 or more or about 0.1 or less, and most preferably at least about 20 or more or about 0.05 or less. The term "about" in this context refers to +/- 5% of a given measurement.

In the case of an odds ratio, a value of 1 indicates that a positive result is equally likely among subjects in both the "diseased" and "control" groups; a value greater than 1 indicates that a positive result is more likely in the diseased group; and a value less than 1 indicates that a positive result is more likely in the control group. In certain preferred embodiments, markers and/or marker panels are preferably selected to exhibit an odds ratio of at least about 2 or more or about 0.5 or less, more preferably at least about 3 or more or about 0.33 or less, still more preferably at least about 4 or more or about 0.25 or less, even more preferably at least about 5 or more or about 0.2 or less, and most preferably at least about 10 or more or about 0.1 or less. The term "about" in this context refers to +/- 5% of a given measurement.

In the case of a hazard ratio, a value of 1 indicates that the relative risk of an endpoint (e.g., death) is equal in both the "diseased" and "control" groups; a value greater than 1 indicates that the risk is greater in the diseased group; and a value less than 1 indicates that the risk is greater in the control group. In certain preferred embodiments, markers and/or marker panels are preferably selected to exhibit a hazard ratio of at least about 1.1 or more or about 0.91 or less, more preferably at least about 1.25 or more or about 0.8 or less, still more preferably at least about 1.5 or more or about 0.67 or less, even more preferably at least about 2 or more or about 0.5 or less, and most preferably at least about 2.5 or more or about 0.4 or less. The term "about" in this context refers to +/- 5% of a given measurement.

In a preferred embodiment of the present invention:
(i) measured amounts of sFlT1 and the cardiac troponin equal or larger than their respective reference amounts indicate a response of the tumor to treatment and cardiotoxic side-effects of the HER-2-inhibitor;
(ii)A measured amount of sFlT1 equal or larger than the reference amount and a measured amount of the cardiac troponin lower than the reference amount indicate a response of the tumor to treatment without cardiotoxic side effects of the HER-2-inhibitor; and
(iii)A measured amount of sFlT1 lower than the reference amount and a measured amount of the cardiac troponin equal or larger than the reference amount indicate cardiotoxic side-effects of the HER-2-inhibitor in the patient and a lacking response of the tumor to treatment.

The reference value for troponin T is, preferably, about 4.0 pg/ml, about 4.5 pg/ml, about 5.0 pg/ml, about 5.5 pg/ml, about 6.0 pg/ml, about 6.5 pg/ml, about 7.0 pg/ml, about 7.5 pg/ml or about 8.0 pg/ml. More preferably, the reference amount is about 6.0 pg/ml. The term "about" in this context refers to +/- 5% of a given measurement.

The reference amount for sFlT1 is, preferably, about 80 pg/ml, about 90 pg/ml, about 100 pg/ml, about 110 pg/ml, about 120 pg/ml, about 130 pg/ml or about 140 pg/ml. More preferably, the reference amount is about 110 pg/ml. The term "about" in this context refers to +/- 5% of a given measurement.

Also preferably, the reference amount for the cardiac troponin and sFlT1 is derived from the amounts of the aforementioned biomarkers determined in a sample that was taken from the patient in question before the onset of the therapy with a HER-2-inhibitor. The amount of the biomarker measured before the onset of therapy is defined as 100 %. Preferably, the reference amount is about 110%, about 120 %, about 130 % or about 140 % of the amount of the cardiac troponin or sFlT1 measured in a sample of the patient taken before the onset of the therapy with the HER-2-inhibitor. Because, typically, the amounts cardiac troponins and sFlT1 in different samples from the same stable patient do not vary by more than 20 %, the reference amount is, more preferably, 120 % of the amount of the respective marker determined before the onset of therapy.

Thus, two kinds of reference amounts can be used according to the method of the present invention. An absolute reference amount can be derived from a group of patients undergoing therapy with a HER-2-inhibitor. Additionally the amounts of the markers of the present invention measured before and after the onset of therapy with a HER-2-inhibitor in the same patient can be used as reference amounts for the method of the present invention.

Preferably, the assessment about success or side effects of the therapy with a HER-2-inhibitor is made based on the amounts of the markers of the present invention in at least one sample.

Preferably, the sample for the determination of the reference amount is taken not more than about 14 days, not more than about 12 days, not more than about 10 days, not more than about 8 days, not more than about 6 days, not more than about 4 days or not more than about 2 days before the onset of therapy with a HER-2-inhibitor.

If the method of the present invention indicates a response of the tumor to the therapy with the HER-2-inhibitor without undesired side effects, the therapy is, preferably, continued.

If the method of the present invention indicates a response of the tumor to therapy with a HER-2-inhibitor combined with undesired side effects, the interval between the samplings should be shortened to less than about 1 week or less than about 2 weeks. Additionally, cardiac function should be monitored with imaging methods or electrocardiography. Preferred imaging methods for the monitoring of cardiac function are echocardiography, computed tomography and magnetic resonance imaging. The aforementioned methods may be combined with stress testing. Preferably, the therapy is continued as long as the cardiac function does not drop below a critical threshold where the life of the patient is in immediate danger. If the cardiotoxic side-effects are pronounced to the extent that they put the patient at risk of severe persisting disorders or death, therapy with the HER-2-inhibitor is, preferably, discontinued or the dosage of the HER-2-inhibitor is reduced. Therapy may be resumed or the dosage of the HER-2-inhibitor may be increased, once the undesired side effects of the HER-2-inhibitor remit. In the case of cardiotoxic side effects, a threshold value for cardiac function may be defined. If the cardiac function rises above this level, therapy with the HER-2-inhibitor can be resumed or the dosage of the HER-2-inhibitor may be increased.

If the method of the present invention indicates that therapy with the HER-2-inhibitor is neither accompanied by undesired side-effects nor by a response of the tumor, the dosage of the HER-2-inhibitor may be increased. Also preferably, the response of the tumor is monitored closely with imaging methods including PET-CT. Switching to alternative therapies may also be considered.

Advantageously, the method of the present invention provides a quick method for assessing or monitoring of the therapy with a HER-2-inhibitor with respect to its success and the possible undesired side effects. The method of the present invention does not require a skilled clinician and can be augmented by automation. Thus, it supplements traditional methods for monitoring based on a clinical examination and allows the selection of those patient that require closer examination due to a possible failure of therapy with a HER-2-inhibitor or due to possible undesired side-effects. Thus, the resources of the health system can be concentrated on the patients in greatest need thereof, while other patients are spared the trouble of unnecessary examinations.

Elevated sFlT1 levels may indicate a response of the tumor to treatment as well as cardiotoxic side-effects of herceptin. Therefore, troponin T is used as a secondary marker in order to detect cardiotoxicity. Based on the measured amounts of troponin T patients with elevated sFlT1 can be diagnosed as suffering from cardiotoxc side-effects of herceptin. This diagnosis can be strengthened by using NT-proBNP as an additional marker for cardiac function.

Therefore, in a preferred embodiment of the present invention the amount of a natriuretic peptide or a variant thereof is determined in step a) of the method of the present invention and the measured amount of the natriuretic peptide or the variant thereof is in step b) compared to a reference amount, wherein an increased amount of the natriuretic peptide indicates cardiotoxic side-effects of the therapy with the HER-2-inhibitor.

The reference amount for the natriuretic peptide or the variant thereof is, preferably, the amount of the natriuretic peptide measured in a sample of the patient taken before the onset of the therapy with the HER-2-inhibitor. Preferably, the sample for the determination of the reference amount is taken not more than about 14 days, not more than about 12 days, not more than about 10 days, not more than about 8 days, not more than about 6 days, not more than about 4 days or not more than about 2 days before the onset of therapy with a HER-2-inhibitor.

A measured amount of the natriuretic peptide that is at least about 10 %, at least about 20 %, at least about 30 % or at least about 40 % higher than the reference amount is, preferably, indicative for cardiotoxic side effects of the therapy with a HER-2-inhibitor.

The term "natriuretic peptide" comprises Atrial Natriuretic Peptide (ANP)-type and Brain Natriuretic Peptide (BNP)-type peptides and variants thereof having the same predictive potential. Natriuretic peptides according to the present invention comprise ANP-type and BNP-type peptides and variants thereof (see e.g. Bonow, 1996, Circulation 93: 1946-1950). ANP-type peptides comprise pre-proANP, proANP, NT-proANP, and ANP. BNP-type peptides comprise pre-proBNP, proBNP, NT-proBNP, and BNP. The pre-pro peptide (134 amino acids in the case of pre-proBNP) comprises a short signal peptide, which is enzymatically cleaved off to release the pro peptide (108 amino acids in the case of proBNP). The pro peptide is further cleaved into an N-terminal pro peptide (NT-pro peptide, 76 amino acids in case of NT-proBNP) and the active hormone (32 amino acids in the case of BNP, 28 amino acids in the case of ANP). ANP and BNP have a vasodilatory effect and cause excretion of water and sodium via the urinary tract. Preferably, natriuretic peptides according to the present invention are NT-proANP, ANP, and, more preferably, NT-proBNP, BNP, and variants thereof. ANP and BNP are the active hormones and have a shorter half-life than their respective inactive counterparts, NT-proANP and NT-proBNP. BNP is metabolised in the blood, whereas NT-proBNP circulates in the blood as an intact molecule and as such is eliminated renally. The in-vivo half-life of NTproBNP is 120 min longer than that of BNP, which is 20 min (Smith 2000, J Endocrinol. 167: 239-46.). Preanalytics are more robust with NT-proBNP allowing easy transportation of the sample to a central laboratory (Mueller 2004, Clin Chem Lab Med 42: 942-4.). Blood samples can be stored at room temperature for several days or may be mailed or shipped without recovery loss. In contrast, storage of BNP for 48 hours at room temperature or at 4° Celsius leads to a concentration loss of at least 20 % (Mueller loc.cit.; Wu 2004, Clin Chem 50: 867-73.). Therefore, depending on the time-course or properties of interest, either measurement of the active or the inactive forms of the natriuretic peptide can be advantageous.

The most preferred natriuretic peptides according to the present invention are NT-proBNP or variants thereof. As briefly discussed above, the human NT-proBNP, as referred to in accordance with the present invention, is a polypeptide comprising, preferably, 76 amino acids in length corresponding to the N-terminal portion of the human NT-proBNP molecule. The structure of the human BNP and NT-proBNP has been described already in detail in the prior art, e.g., WO 02/089657, WO 02/083913 or Bonow loc. cit. Preferably, human NT-proBNP as used herein is human NT-proBNP as disclosed in EP 0 648 228 B1. These prior art documents are herewith incorporated by reference with respect to the specific sequences of NT-proBNP and variants thereof disclosed therein. The NT-proBNP referred to in accordance with the present invention further encompasses allelic and other variants of said specific sequence for human NT-proBNP discussed above. Specifically, envisaged are variant polypeptides which are on the amino acid level preferably, at least 50%, 60%, 70%, 80%, 85%, 90%, 92%, 95%, 97%, 98%, or 99% identical, to human NT-proBNP, preferably over the entire length of human NT-proBNP. The degree of identity between two amino acid sequences can be determined by algorithms well known in the art. Preferably, the degree of identity is to be determined by comparing two optimally aligned sequences over a comparison window, where the fragment of amino acid sequence in the comparison window may comprise additions or deletions (e.g., gaps or overhangs) as compared to the reference sequence (which does not comprise additions or deletions) for optimal alignment. The percentage is calculated by determining the number of positions at which the identical amino acid residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith and Waterman Add. APL. Math. 2:482 (1981), by the homology alignment algorithm of Needleman and Wunsch J. Mol. Biol. 48:443 (1970), by the search for similarity method of Pearson and Lipman Proc. Natl. Acad Sci. (USA) 85: 2444 (1988), by computerized implementations of these algorithms (GAP, BESTFIT, BLAST, PASTA, and TFASTA in the Wisconsin Genetics Software Package, Genetics Computer Group (GCG), 575 Science Dr., Madison, WI), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used. Variants referred to above may be allelic variants or any other species specific homologs, paralogs, or orthologs. Substantially similar and also envisaged are proteolytic degradation products which are still recognized by the diagnostic means or by ligands directed against the respective full-length peptide. Also encompassed are variant polypeptides having amino acid deletions, substitutions, and/or additions compared to the amino acid sequence of human NT-proBNP as long as the said polypeptides have NT-proBNP properties. NT-proBNP properties as referred to herein are immunological and/or biological properties. Preferably, the NT-proBNP variants have immunological properties (i.e. epitope composition) comparable to those of NT-proBNP. Thus, the variants shall be recognizable by the aforementioned means or ligands used for determination of the amount of the natriuretic peptides. Biological and/or immunological NT-proBNP properties can be detected by the assay described in Karl et al. (Karl 1999, Scand J Clin Invest 230:177-181), Yeo et al. (Yeo 2003, Clinica Chimica Acta 338:107-115). Preferred properties of ANP and BNP are their vasodilatory effect and the induction of the excretion of water and sodium via the urinary tract. Variants also include posttranslationally modified peptides such as glycosylated peptides. Further, a variant in accordance with the present invention is also a peptide or polypeptide which has been modified after collection of the sample, for example by covalent or non-covalent attachment of a label, particularly a radioactive or fluorescent label, to the peptide.

Moreover, the present invention relates to a method for the diagnosis of an acute cardiovascular event based on the determination of a cardiac troponin and sFlT1 in a sample of a patient.

The method of the present invention, preferably, comprises the steps of a) determining the amounts of sFlT1 and a cardiac troponin in a sample of a patient; b) comparing the measured amounts of the cardiac troponin and sFlT1 with reference amounts; and c) diagnosing the acute cardiovascular event based on the results of the comparison in step b).

Furthermore, the present invention relates to a device for assessing the effects of a therapy with a HER-2-inhibitor in a patient comprising
a) an analyzing unit for determining the amounts of sFlt1 or a variant thereof and a cardiac troponin or a variant thereof in a sample of a patient; and
b) an evaluation unit for comparing the measured amounts of sFlt1 or the variant thereof and the cardiac troponin or the variant thereof with reference amounts.

The term "device" as used herein relates to a system of means comprising at least the aforementioned means operatively linked to each other as to practice the method of the present invention. Preferred means for determining the amounts of the markers of the present invention, and means for carrying out the comparison are disclosed above in connection with the method of the invention. How to link the means in an operating manner will depend on the type of means included into the device. For example, where an analysis unit for automatically determining the amount of the gene products of the present invention is applied, the data obtained by said automatically operating analysis unit can be processed by, e.g., a computer as evaluation unit in order to obtain the desired results. Preferably, the means are comprised by a single device in such a case.

Said device, preferably, includes an analyzing unit for the measurement of the amount of a cardiac troponin or a variant thereof and the amount of sFlT1 or a variant thereof in an applied sample and an evaluation unit for processing the resulting data. Preferably, the evaluation unit comprises a database with the stored reference amounts and a computer program code which when tangibly embedded on a computer carries out the comparison of the determined amounts and the reference amounts stored in the database. More preferably, the evaluation unit comprises a further computer program code which allocates the result of the comparison to a risk prediction. In such a case, it is, also preferably, envisaged that the evaluation unit comprises a further database wherein the reference amounts are allocated to the risks.

Alternatively, where means such as test stripes are used for determining the amount of the cardiac troponin or the variant thereof and the amount of sFlT1 or the variant thereof, the evaluation unit may comprise control stripes or tables allocating the determined amount to a reference amount. The test stripes are, preferably, coupled to ligands which specifically bind to the cardiac troponin and sFlT1. The strip or device, preferably, comprises means for detection of the binding of said cardiac troponin and sFlT1 to said ligands. Preferred means for detection are disclosed in connection with embodiments relating to the method of the invention above. In such a case, the analysis unit and the evaluation unit are operatively linked in that the user of the system brings together the result of the determination of the amount and the diagnostic or prognostic value thereof due to the instructions and interpretations given in a manual. The analysis unit and the evaluation unit may appear as separate devices in such an embodiment and are, preferably, packaged together as a kit. The person skilled in the art will realize how to link the means without further ado. Preferred devices are those which can be applied without the particular knowledge of a specialized clinician, e.g., test stripes or electronic devices which merely require loading with a sample. The results may be given as output of raw data which need interpretation by the clinician. Preferably, the output of the device is, however, processed, i.e. evaluated, raw data the interpretation of which does not require a clinician. Further preferred devices comprise the analyzing units/devices (e.g., biosensors, arrays, solid supports coupled to ligands specifically recognizing the gene product, Plasmon surface resonance devices, NMR spectrometers, mass-spectrometers etc.) or evaluation units/devices referred to above in accordance with the method of the invention.

Preferably, the analyzing unit of the device of the present invention further comprises means for determining the amount of a natriuretic peptide in a sample of the patient and the evaluation unit further comprises means for comparing the measured amount of the natriuretic peptide with a suitable reference amount.

Finally, the present invention relates to a kit for assessing the effects of a therapy with a HER-2-inhibitor in a patient comprising
a) an analyzing agent for determining the amounts of sFlt1 or a variant thereof and a cardiac troponin or a variant thereof in a sample of a patient; and
b) an evaluation unit for comparing the measured amounts of sFlt1 or the variant thereof and the cardiac troponin or the variant thereof with reference amounts.

The term "kit" as used herein refers to a collection of the aforementioned components of which may or may not be packaged together. The components of the kit may be comprised by separate vials (i.e. as a kit of separate parts) or provided in a single vial. Moreover, it is to be understood that the kit of the present invention is to be used for practising the methods referred to herein above. It is, preferably, envisaged that all components are provided in a ready-to-use manner for practising the methods referred to above. Further, the kit preferably contains instructions for carrying out the said methods. The instructions can be provided by a user's manual in paper- or electronic form. For example, the manual may comprise instructions for interpreting the results obtained when carrying out the aforementioned methods using the kit of the present invention. The kit shall comprise an analyzing agent. This agent is capable of specifically recognizing the peptides of the present invention in a sample of the subject. Moreover, the said agent shall upon binding to the peptides of the present invention, preferably, be capable of generating a detectable signal, the intensity of which correlates to the amount of the peptides of the present invention present in the sample. Dependent on the type of signal which is generated, methods for detection of the signal can be applied which are well known in the art. Analyzing agents which are preferably used for the kit of the present invention include antibodies or aptamers. The analyzing agent may be present on a test stripe as described elsewhere herein. The amounts of the peptides of the present invention thus detected can then be further evaluated in the evaluation unit. Preferred evaluation units to e used for the kit of the present invention include those referred to elsewhere herein.

Preferably the analyzing agent of the kit of the present invention further comprises means for determining the amount of a natriuretic peptide in a sample of the patient and the evaluation unit further comprises means for comparing the measured amount of the natriuretic peptide with a reference amount.

The present invention also relates to the use of a kit or device for determining the amount of sFLT-1 or a variant thereof and a cardiac troponin or a variant thereof and optionally a natriuretic peptide or a variant thereof in a sample of a subject, comprising means for determining the amount of sFLT-1 or the variant thereof and the cardiac troponin or the variant thereof and optionally the natriuretic peptide or the variant thereof and/or means for comparing the amount of sFLT-1 or the variant thereof and the cardiac troponin or the variant thereof and optionally the natriuretic peptide or the variant thereof to at least one reference amount for: monitoring the therapy with a HER-2-inhibitor or for assessing its effects.

The present invention also relates to the use of: an antibody against a cardiac troponin or a variant thereof and an antibody against sFLT-1 or a variant thereof and optionally a an antibody against natriuretic peptide or a variant thereof and/or of means for determining the amount of sFLT-1 or a variant thereof and a cardiac troponin or a variant thereof and optionally a natriuretic peptide or a variant thereof and/or of means for comparing the amount of the cardiac troponin or the variant thereof and sFLT-1 or the variant thereof and optionally the natriuretic peptide or the variant thereof to at least one reference amount for the manufacture of a diagnostic composition for: monitoring the therapy with a HER-2-inhibitor or for assessing its effects.

The present invention also relates to the use of: an antibody against sFLT-1 or a variant thereof and an antibody against a cardiac troponin or a variant thereof and optionally an antibody against a natriuretic peptide or a variant thereof and/or of means for determining the amount of a sFLT-1 or a variant thereof and a cardiac troponin or a variant thereof, and optionally a natriuretic peptide or a variant thereof and/or of means for comparing the amount of sFLT-1 or the variant thereof and of the cardiac troponin or the variant thereof and optionally of the natriuretic peptide or the variant thereof to at least one reference amount for: monitoring the therapy with a HER-2-inhibitor or for assessing its effects.

Accordingly, the present invention relates, furthermore, to a method for the diagnosis of an acute cardiovascular event comprising the steps of
a) determining the amounts of sFlT1 and a cardiac troponin in a sample of a patient;
b) comparing the measured amounts of the cardiac troponin and sFlT1 with reference amounts; and
c) diagnosing the acute cardiovascular event based on the results of the comparison in step b).

The following examples are merely intended to illustrate the present invention. They shall not limit the scope of the claims in any way.

### Example 1: Analytical Methods

sFlt-1 was determined with a sFlt-1 immunoassay to be used with the Elecsys and COBAS analyzers from Roche Diagnostics, Mannheim, Germany. The assay is based on the sandwich principle and comprises two monoclonal sFlt-1 specific antibodies. The first of these is biotinylated and the second one is labeled with a Tris(2,2'-hipyridyl)ruthenium(TT)-complex. In a first incubation step both antibodies are incubated with the human serum sample. A sandwich complex comprising sFlt-1 and the two different antibodies is formed. In a next incubation step streptavidin-coated beads are added to this complex. The beads bind to the sandwich complexes. The reaction mixture is then aspirated into a measuring cell where the beads are magnetically captured on the surface of an electrode. The application of a voltage then induces a chemiluminescent emission from the ruthenium complex which is measured by a photomultiplier. The amount of light is dependent on the amount of sandwich complexes on the electrode. The test is capable of measuring sFlT1-concentrations from 10 to 85000 pg/ml.

Troponin T was measured with an immunoassay to be used with the above described systems. The test is capable of measuring troponin T-concentrations from 1 to 25000 pg/ml.

Serum levels of NT-proBP were determined by a immunoassay to be used with the Elecsys and COBAS analyzers from Roche Diagnostics. The principle underlying the test is the same as for the sFlT1 test. The test is capable of measuring NT-proBNP-concentrations from 5 to 35000 pg/ml.

### Example 2: Troponin T and sFlT1 in healthy subjects

149 subjects, mean age 42.8 years were tested for the presence of sFlT1 and sensitive troponin T. All individuals were clinically healthy and did not have risk factors of cardiovascular disease such as smoking, elevated lipid levels or diabetes and had normal kidney function as assessed by creatinine in serum and results within the normal range of the test.

| Table 1: Troponin T and sF1T1 in apparently healthy subjects | | |
|---|---|---|
| Percentile | sF1T1 [pg/ml] | TroponinT [pg/ml] |
| 25^{th} | 42 | 0* |
| 50^{th} | 52 | 0* |
| 75^{th} | 71 | 0* |
| 90^{th} | 81 | 2.0 |
| 95^{th} | 92 | 2.95 |
| 99^{th} | 109 | 4.5 |

| | | |
|---|---|---|
| *below the limit of detection | | |

### Example 3: Troponin T and sFlT1 in patients with stable coronary artery disease

Tumor patients may -especially at increased age - have coronary artery disease (CAD) in addition to their cancer disease. CAD may be asymptomatic and not recognized clinically. Thus, a second control group consisted of patients with established coronary artery disease. The control group comprised 234 patients (122 males and 112 females, mean age 52.1 years). All patients were clinically stable at least 3 months prior to study entry. Particularly, they had no signs and symptoms of chest pain or even acute coronary syndrome or dyspnoe. 82 patients had one vessel disease, 60 patients had two vessel disease and 92 patients had three vessel disease as determined by coronary angiography. Vessel disease was defined as at least 50 % narrowing of a coronary artery at at least one site. All patients had creatinine levels in the normal range.

| Table 2: Troponin T and sF1T1 I patients with coronary artery disease | | |
|---|---|---|
| Percentile | sF1T1 [pg/ml] | TroponinT [pg/ml] |
| 25^{th} | 75 | 1.24 |
| 50^{th} | 95 | 6.6 |
| 75^{th} | 124 | 23.0 |
| 95^{th} | 184 | 82.0 |

Based on the data shown in tables 1 and 2 and considering patients with clinically apparent cardiovascular disease (scheduled for angiography to confirm or exclude CAD) a reference amount of 110 pg/ml was chosen for sFlT1 and a reference amount of 6.0 pg ml was chosen for troponin T.

### Example 4: Troponin T, sFlT1 and NT-proBNP in patients receiving trastuzumab

A total of 17 women (mean age 48.9 years) and with a diagnosis of advanced breast cancer and without clinical evidence of coronary artery disease were included into the study to receive herceptin (trastuzumab) therapy. All patients had normal kidney function. In total 61 samples were available which included 1 sample from each patient obtained before treatment. Patients did not complain about chest pain before or during treatment. In none of the patients therapy was discontinued during the observation period of in total 4 months after start of treatment.

| Table 3: sF1T1 and cardiac troponin T in patients undergoing herceptin-therapy | | | | |
|---|---|---|---|---|
| | N above reference value | N increase from baseline² | Tumor response³ | Cardiotoxic side-effects⁴ |
| Troponin T and sFlT1¹ | 7 | 7 | 5 | 6 |
| Troponin T¹ | 6 | 4 | 0 | 2 |
| SFlT1¹ | 4 | 4 | 3 | 0 |

| | | | | |
|---|---|---|---|---|
| ¹Number of patients under herceptin-therapy with at least one sample above the reference value for the respective marker(s) ²Increase of the respective markers in at least one sample taken during herceptin-therapy by at least 20 % as compared to the sample taken before treatment ³Size reduction of the tumor by at least 50 % ⁴As indicated by an increase of Troponin T from pretreatment level of at least 20 % with and without concomitant increase in NT-pro BNP | | | | |

In the group of patients with troponin T and sFlT1 above the reference value both markers showed additionally an increase of at least 20 % as compared to a sample taken before the onset of herceptin-therapy. In stable patients the amounts of cardiac troponin T and sFlT1 do not vary by more than 20%. This indicates that the high amounts of troponin T and sFlT1 were caused by the herceptin therapy and not by preexisting cardiac disease. In 6 of these patients BNP-levels were increased after herceptin-therapy indicating impaired cardiac function.

In the group of patients with troponin T levels above the reference value but normal sFlt1 only 4 out of 6 patients showed an increase of troponin T as compared to the amount determined before herceptin-therapy. Therefore, the two patients without therapy-induced troponin increase are considered to have suffered from a cardiac disease prior to therapy.

If only sFlT1 was higher than the reference amount, 3 out of 4 patients showed a tumor response.

In the present study only two patients with an increased level of NT-proBNP had dyspnoe after exercise. None of the patients in this study showed clinical evidence for rales, S3 (gallop rhythm with a third heart sound) or jugular distension, the latter being signs of moderate to severe heart failure. Thus, worsening of cardiac function was mild in all cases studied.

It could be shown that the comparison of troponin T or NT-proBNP to an absolute reference amount derived from subjects with or without cardiac disease gives almost the same information as the comparison of the markers in a sample taken after the onset of therapy to the levels measured in a sample from the same patient measured before the onset of therapy.

Thanks to the measurement of sFlT1, ischemic processes in patients suffering from cancer and treated with HER2 inhibitors can be detected. Tumor patients frequently suffer from cardiac disorders, specifically atherosclerosis because the incidence of cancer as well as of cardiac disorders increases with age. Ischemic processes are not tissue specific and may indicate beneficial effects such as tumor necrosis or unwanted effects, particularly cardiac ischemia. The additional determination of the cardiac specific protein Troponin reflecting cardiac necrosis and a natriuretic peptide reflecting cardiac function allows to dissect the origin and nature of the ischemia, thus allowing appropriate clinical consequences such as more intense monitoring both using laboratory tests and imaging methods including targeted imaging, modification of drug therapy or even complete discontinuation of therapy or treatment interruption.

## Claims

1. A method for assessing the effects of a therapy with a HER-2-inhibitor in a patient comprising the steps of
a) determining the amount of sFlt-1 or a variant thereof and a cardiac troponin or a variant thereof in a sample of the patient;
b) comparing the measured amounts of sFIT-1 or the variant thereof and the cardiac troponin or the variant thereof with reference amounts; and
c) assessing the effects of the therapy with a HER-2-inhibitor based on the results obtained in step b).

2. The method of claim 1, wherein the cardiac troponin is troponin T.

3. The method of claim 2, wherein the reference amount for troponin T is 6.0 pg/ml.

4. The method of claim 2, wherein the reference amount for troponin T is 120 % of the amount of troponin T measured in the patient before the onset of treatment with the ERB2-inhibitor.

5. The method of any of claims 1 to 4, wherein the reference amount for sFlT1 is 110 pg/ml.

6. The method of any of claims 1 to 4, wherein the reference amount for sFlT1 is 120 % of the amount of sFlT1 measured in the patient before the onset of treatment with the ERB2-inhibitor.

7. The method of any of claims 1 to 6, wherein a natriuretic peptide or a variant thereof is additionally determined in step a) and a measured amount of the natriuretic peptide higher than a reference amount indicates cardiotoxic side-effects of the HER-2-inhibitor in the patient.

8. The method of claim 7, wherein the reference amount for the natriuretic peptide is 120 % of the amount of the natriuretic peptide measured in the patient before the onset of treatment with the HER-2-inhibitor

9. The method of claim 7 or 8, wherein the natriuretic peptide is NT-proBNP.

10. The method of any of claims 1 to 9, wherein the HER-2-inhibitor is trastuzumab.

11. A method for monitoring the therapy with a HER-2-inhibitor in a patient comprising the steps of
a) repeatedly determining the amount of sFlt-1 or a variant thereof and a cardiac troponin or a variant thereof in more than one sample;
b) comparing the amounts of sFIT-1 or the variant thereof and the cardiac troponin or the variant thereof measured according to step a) to reference amounts; and
c) deciding whether the therapy with a HER-2-inhibitor achieves the desired effects and/or causes undesired side-effects based on the comparison of step b).

12. A device for assessing the effects of a therapy with a HER-2-inhibitor in a patient comprising
a) an analyzing unit for determining the amounts of sFlt1 or a variant thereof and a cardiac troponin or a variant thereof in a sample of a patient; and
b) an evaluation unit for comparing the measured amounts of sFlt1 or the variant thereof and the cardiac troponin or the variant thereof with reference amounts.

13. The device of claim 12, wherein the analyzing unit further comprises means for determining the amount of a natriuretic peptide in a sample of the patient and the evaluation unit further comprises means for comparing the measured amount of the natriuretic peptide with a suitable reference amount.

14. A kit for assessing the effects of a therapy with a HER-2-inhibitor in a patient comprising
a) an analyzing agent for determining the amounts of sFlt1 or a variant thereof and a cardiac troponin or a variant thereof in a sample of a patient; and
b) an evaluation unit for comparing the measured amounts of sFlt1 or the variant thereof and the cardiac troponin or the variant thereof with reference amounts.

15. The kit of claim 14, wherein the analyzing agent further comprises means for determining the amount of a natriuretic peptide in a sample of the patient and the evaluation unit further comprises means for comparing the measured amount of the natriuretic peptide with a reference amount.
